(19) Europäisches Patentamt
European Patent Office
Office européen des brevets

(11) Numéro de publication: **0 247 931**
**A1**

(12) # DEMANDE DE BREVET EUROPEEN

(21) Numéro de dépôt: **87401155.4**

(22) Date de dépôt: **22.05.87**

(51) Int. Cl.4: **C 12 M 1/40**

(30) Priorité: **30.05.86 FR 8607792**

(43) Date de publication de la demande:
**02.12.87 Bulletin 87/49**

(84) Etats contractants désignés:
**AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75007 Paris (FR)**

(72) Inventeur: **Alexandre, Stéphane, Jacques**
**8 rue des Camélias**
**F-76800 Saint-Etienne-du-Rouvray (FR)**

**Thellier, Michel**
**240, rue des Champs Preaux**
**F-76160 Darnetal (FR)**

**Vincent, Jean-Claude**
**F-76690 Cleres (FR)**

(74) Mandataire: **Ores, Irène et al**
**CABINET ORES 6, Avenue de Messine**
**F-75008 Paris (FR)**

(54) **Procédé d'extraction de composés à haute valeur ajoutée à partir de solutions complexes et dispositif membranaire pour la mise en oeuvre de ce procédé.**

(57) Procédé et dispositif pour l'extraction sélective de composés, notamment biochimiques, à haute valeur ajoutée, à partir d'une solution complexe.

Le procédé utilise un système réactif membranaire, (3), (4), (5), (6) monoenzymatique placé en milieu liquide, aux interfaces duquel un gradient de potentiel électrochimique de l'un des réactifs crée des conditions asymétriques qui forcent la réaction réversible catalysée par le système réactif membranaire, à travailler dans des sens opposés de part et d'autre dudit système, le passage du composé à extraire d'un côté vers l'autre étant favorisé par la présence sur les deux faces dudit système,de barrières (5) de confinement d'un composé autre que le composé à extraire. Dispositif pour la mise en oeuvre de ce procédé.

Application à l'extraction de composés intéressants du point de vue biochimique, par concentration et accumulation d'un côté du système membranaire.

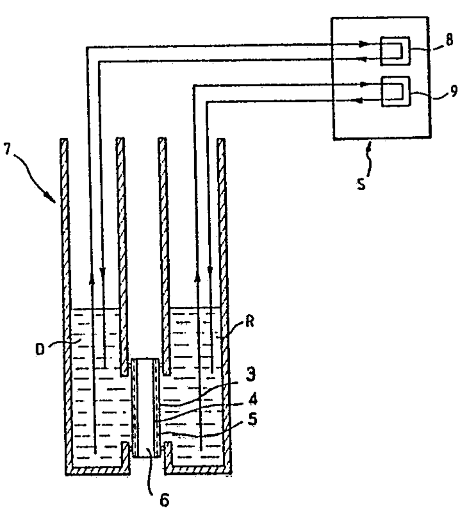


FIG. 3

EP 0 247 931 A1

## Description

PROCEDE D'EXTRACTION DE COMPOSES A HAUTE VALEUR AJOUTEE A PARTIR DE SOLUTIONS COMPLEXES ET DISPOSITIF MEMBRANAIRE POUR LA MISE EN OEUVRE DE CE PROCEDE.

La présente invention est relative à un procédé d'extraction de composés à haute valeur ajoutée, et en particulier de composés biochimiques, à partir de solutions complexes et à un dispositif pourvu d'une membrane réactive, propre à la mise en oeuvre de ce procédé.

En biologie, de nombreux passages trans-membranaires de molécules ou d'ions se font avec une cinétique à saturation et sont attribués à un mécanisme enzymatique de type perméase. Lorsque le système membranaire est capable de faire passer la molécule ou l'ion transporté d'un côté où il se trouve à faible potentiel électrochimique à l'autre côté où son potentiel électrochimique est plus élevé, le mécanisme qui constitue un transport actif nécessite la présence d'une asymétrie. C'est ainsi que l'étude de certaines pompes ioniques ou moléculaires a montré une localisation asymétrique des sites réactionnels au sein des membranes, que ce soit dans l'asymétrie structurale d'une protéine, comme par exemple son organisation en sous-unités, certaines de ces sous-unités étant sur la face interne de la membrane, d'autres sur la face externe, comme pour l'ATPase, ou que ce soit dans l'asymétrie structurale du système transporteur, qui peut être multienzymatique ou comporter plusieurs complexes protéiques ou autres localisés à des endroits bien spécifiques de la membrane comme dans la chaine de la respiration, les photo-systèmes, etc...

L'étude in vivo de ces mécanismes reste toujours d'une grande complexité. La modélisation, en utilisant des systèmes artificiels plus simples dans lesquels le nombre des paramètres est limité, permet d'approcher les phénomènes biologiques complexes, tout en réalisant des transports actifs d'ions ou de molécules.

C'est ainsi qu'il a été proposé dans l'Art antérieur, des modèles de transport actif dans lesquels l'asymétrie nécessaire au transport vectoriel est structurale, c'est-à-dire qu'elle réalise une répartition asymétrique des molécules d'enzyme dans la membrane [ cf THOMAS & CAPLAN, "Membrane Separation Processes", Ed. MEARES, ELSEVIER, Amsterdam, Pays-Bas (1976), 351 ] .

L'un des Inventeurs a montré qu'il est possible de remplacer cette asymétrie structurale permanente par une asymétrie fonctionnelle dans laquelle les réactions sont activées ou inhibées par un effecteur [ (J.C. VINCENT, Thèse de Doctorat ès Sciences, Rouen, France (1980) ] ; cette asymétrie fonctionnelle a été démontrée dans un modèle constitué par une membrane à deux enzymes distribuées uniformément, dans laquelle les fonctions enzymatiques sont réparties dans l'espace, en introduisant et en maintenant un gradient de pH sur la membrane [ (SELEGNY et VINCENT, BIOPHYS. CHEM. 12 (1980) 93-106 et 12 (1980) 107-113)] . Une publication du Colloque du CNRS No 258 (1976) sur les ECHANGES IONIQUES TRANSMEMBRANAIRES CHEZ LES VEGETAUX intitulée "ACTIVE TRANSPORT OF GLUCOSE IN VITRO, ILLUSTRATION OF ASYMMETRICAL FUNCTIONAL STRUCTURES AND OF ALLOTOPIA" parue sous la signature de J.C. VINCENT, E. SELEGNY et Y. COMBRET, décrit une membrane de transport actif de glucose présentant ce type d'asymétrie fonctionnelle. Cette membrane active est obtenue en coulant un gel d'agarose contenant de l'hexokinase et de la phosphatase acide en un film qui est traité par le glutaraldéhyde et qui contient donc les enzymes immobilisées, réparties de façon homogène. Sur les côtés de la membrane active sont placées des structures constituant des barrières portant des charges négatives (lesquelles "barrières" sont dénommées "valves" dans cette publication), qui sont en agarose contenant de l'acide polyacrylique et qui ont pour rôle d'éviter la diffusion d'ions glucose-phosphate chargés, à travers la membrane active [ cf également l'Article intitulé "TWO ENZYME ACTIVE TRANSPORT IN VITRO WITH PH-INDUCED ASYMMETRICAL FUNCTIONAL STRUCTURES - I - THE MODEL AND ITS ANALYTICAL TREATMENT" de E. SELEGNY et J.C. VINCENT dans BIOPHYSICAL CHEMISTRY 12 (1980) 93-106] .

Le principe du modèle de transport actif repose sur l'existence d'un cycle réactionnel plurienzymatique dont le plus court est un système bienzymatique du type

$$S \xrightarrow{E_1} P \text{ et } P \xrightarrow{E_2} S.$$

. Les deux enzymes $E_1$ et $E_2$ du cycle doivent alors être différemment influencées par un effecteur de sorte que lorsqu'elles sont insérées dans une structure membranaire traversée par un gradient de cet effecteur, leurs activités se distribuent le long de ce gradient, aboutissant à une structure fonctionnelle à deux couches. Dans la première couche, S est consommé, il entre donc par diffusion du compartiment donneur D vers la membrane et est transformé en P par $E_1$, le produit P diffusant dans la membrane est à nouveau transformé en S dans la seconde couche d'activité $E_2$ ; l'excès de S est alors éliminé de la membrane par diffusion vers le compartiment receveur R. La stoechiométrie de ce transport actif est assurée par la présence symétrique de "barrières" confinant le composé P à l'espace intramembranaire.

Le principal inconvénient de ce modèle est la nécessité dans laquelle on est de trouver les couples enzymatiques adéquats, le système hexokinase-phosphatase étant un des rares exemples de couples enzymatiques susceptibles de fonctionner conjointement de façon cyclique.

Comme on le sait, la plupart des réactions chimiques sont réversibles : si une réaction implique deux composés A et B, il y a une compétition constante entre les deux réactions élémentaires A→B et B→A et la vitesse de réaction apparente correspond à la différence entre les vitesses des deux réactions élémentaires.

Lorsqu'une enzyme catalyse une réaction chimique, elle augmente simultanément les vitesses des deux réactions élémentaires. Ainsi, les réactions réversibles sont fréquentes en enzymologie. On peut citer, par exemple, les protéases qui, en milieu aqueux, catalysent la décomposition de peptides et qui catalysent également, en milieu non aqueux, la synthèse de peptides. La plupart des déshydrogénases appartiennent à ce groupe et présentent un grand intérêt car les réactions dans les deux sens peuvent avoir lieu dans le même milieu aqueux.

La présente invention est basée sur la combinaison de l'utilisation de la réversibilité de nombreuses réactions catalysées par une seule et même enzyme et de l'application des travaux antérieurs mentionnés plus haut, auxquels a participé l'un des Inventeurs, en réalisant le cycle réactionnel du modèle de transport actif par les deux réactions élémentaires de la réaction réversible et en utilisant un réactif dont le gradient agit directement par la loi d'action de masse sur la réaction, pour réaliser l'asymétrie.

La présente invention se distingue encore des travaux précédemment décrits concernant les modèles bienzymatiques en ce que le procédé ne nécessitant qu'une seule enzyme facilite la fabrication, réduit le coût d'utilisation, élimine les problèmes de compatibilité des molécules enzymatiques entre elles et simplifie les conditions d'utilisation.

La présente invention a pour objet un procédé d'extraction sélective d'un composé à haute valeur ajoutée, et notamment d'un composé biochimique, à partir d'une solution complexe, caractérisé en ce que l'on met en oeuvre pour réaliser ladite extraction, un système réactif membranaire monoenzymatique, apte à catalyser une réaction réversible, qui est placé en milieu liquide, en ce que l'on crée aux interfaces système membranaire/milieu liquide, des conditions asymétriques à l'aide d'un gradient de potentiel électrochimique de l'un des réactifs, lesquelles conditions asymétriques forcent la réaction réversible à travailler dans un sens d'un côté du système réactif membranaire et dans l'autre sens de l'autre côte, en ce que le composé à extraire effectue, par suite, une boucle de réaction qui le fait passer d'un côté à l'autre de la membrane et en ce que le passage du composé à extraire d'un côté du système réactif membranaire vers l'autre côté, et son accumulation de ce côté sont favorisés par la présence sur les deux faces dudit système d'une couche constituant une barrière spécifique propre à confiner dans l'espace intramembranaire au moins l'un des composés autres que le composé à extraire.

On entend par "barrière" une structure qui interdit le passage du composé à confiner dans l'espace intramembranaire ; les moyens d'interdiction du passage sont choisis en fonction de la nature du composé à confiner. En particulier, si le composé à confiner est chargé, ils peuvent être constitués par une charge globale de signe opposé portée par la barrière ; si le composé à confiner est une grosse molécule, ils peuvent être constitués par un film microporeux ; si le composé à confiner présente une hydrophobicité donnée, ils peuvent être constitués par une substance conférant à la barrière une hydrophobicité de nature opposée.

Par voie de conséquence, le rapport des coefficients de diffusion composé à extraire/composé à confiner dans l'espace intramembranaire est $\gg 1$.

Les Inventeurs ont trouvé qu'il est possible d'assurer un transport actif ou facilité d'une molécule en utilisant une seule enzyme au lieu des deux mises en oeuvre jusqu'à maintenant. En effet, dans l'Art antérieur on a cherché à mimer le système biologique en introduisant une asymétrie dans la structure du catalyseur, et plus spécifiquement en créant une asymétrie par action sur les catalyseurs enzymatiques eux-mêmes, soit en répartissant de façon asymétrique les molécules enzymatiques dans les structures membranaires, soit en distribuant leurs activités de façon asymétrique par un gradient d'effecteur (proton ou autre, qui modifie la réaction mais n'y participe pas obligatoirement). Au contraire, conformément à l'invention, les Inventeurs ont trouvé que l'asymétrie recherchée peut être obtenue non pas en agissant sur les catalyseurs enzyma tiques, mais en agissant sur la réaction chimique elle-même, par la loi d'action de masse, c'est-à-dire sur les concentrations des réactifs alors que le catalyseur enzymatique, désormais unique, n'a plus à intervenir pour réaliser l'asymétrie et n'intervient que par sa spécificité et sa localisation membranaire.

Ainsi dans le système membranaire monoenzymatique conforme à l'invention, l'hétérogénéité moléculaire des systèmes à deux enzymes a disparu et ceci va à l'encontre des considérations biomimétiques qui voudraient que l'on conserve, comme dans la matière vivante, une certaine hétérogénéité ou asymétrie structurale moléculaire pour assurer un transport actif.

La présente invention se distingue des travaux précédemment décrits concernant les modèles bienzymatiques en ce que le procédé met en oeuvre une seule enzyme pour réaliser les deux sens de la réaction A⇆B. Ce fait est fondamental car il introduit la notion de réversibilité de la réaction, notion totalement absente des travaux de l'Art antérieur, mais surtout parce qu'il étend considérablement la portée de ce procédé d'extraction :

Le fait de mettre en jeu une seule enzyme présente un avantage considérable car il permet un choix très large parmi les enzymes existantes et permet donc des extractions de molécules très variées, alors que dans les modèles à deux enzymes où chacune catalyse une partie du cycle (A→B puis B→A), la difficulté réside dans le choix très restreint de tels couples d'enzymes acceptant les mêmes substrats, et par voie de conséquence, dans la possibilité très limitée de transports actifs de quelques molécules.

1) Le procédé peut fonctionner dès que l'on a une réaction enzymatique réversible entre au moins trois réactifs, le premier que l'on veut extraire, le deuxième jouant le rôle de réactif auquel on applique un gradient et le troisième que l'on confine à l'espace intramembranaire. Le processus est alors un transport actif.

2) Le procédé peut s'étendre à une réaction enzymatique à au moins deux réactifs, du type A⇄B en

appliquant le gradient de potentiel chimique au composé que l'on veut extraire, lui-même. La source énergétique est alors la dissipation de ce gradient et le processus est un transport facilité, qui va donc augmenter sélectivement la vitesse de passage à travers la membrane,du composé à extraire.

Le procédé permet dans ce cas d'extraire mais non de concentrer l'espèce considérée.

3) Pour d'autres applications, et quel que soit le nombre de réactifs de la réaction, le procédé peut également être utilisé à condition de limiter le processus à la formation et décomposition du complexe enzyme-composé à extraire $E + A \rightleftarrows EA$, l'enzyme elle-même,sous forme libre ou complexée, jouant le rôle de la substance confinée dans la membrane et le gradient pouvant être établi soit en jouant sur l'une des formes de l'enzyme dans la membrane, auquel cas ce gradient peut être obtenu par un gradient d'effecteur et notamment de protons, le processus étant alors un transport actif permettant de concentrer le composé à extraire. soit en jouant sur le composé à extraire lui-même, auquel cas le processus est un transport facilité et on ne peut qu'accélérer sélectivement la diffusion du composé à extraire.

Selon un mode de réalisation avantageux du procédé objet de la présente invention, lorsque la réaction implique au moins trois réactifs, à savoir un composé à extraire, au moins un co-réactif et au moins un composé destiné à être confiné dans l'espace intramembranaire défini par le système membranaire, on établit le gradient de potentiel électrochimique du co-réactif ou de l'un des co-réactifs, pour une constante d'équilibre $K_{eq}$ donnée, en ajustant les potentiels électrochimiques à des valeurs différentes dans les compartiments récepteur et donneur définis par le système membra naire, de part et d'autre de ce dernier, de telle façon que le rapport K donné par la loi d'action de masse, dans lequel la concentration du composé à extraire est en dénominateur, soit

$K < K_{eq}$ du côté donneur et

$K > K_{eq}$ du côté récepteur.

Selon un autre mode de réalisation avantageux du procédé objet de la présente invention, lorsque la réaction implique des protons, on utilise avantageusement ces protons comme réactif dont on veut établir le gradient, en ajustant les compartiments donneur et récepteur situés de part et d'autre du système membranaire, à des pH différents.

Selon encore un autre mode de réalisation avantageux du procédé objet de la présente invention, lorsque la réaction implique deux réactifs, à savoir un composé à extraire et un réactif, on forme un complexe entre le composé à extraire et l'enzyme, on confine à la fois l'enzyme et le complexe enzyme-composé dans l'espace intramembranaire et on établit un gradient intermédiaire d'un effecteur approprié, tel, notamment qu'un gradient de pH, lequel gradient intermédiaire provoque dans l'espace intramembranaire, l'établissement d'un gradient d'enzyme libre active, qui est le gradient de potentiel électrochimique voulu.

Conformément aux lois de la diffusion, la vitesse d'extraction du système membranaire est inversement proportionnelle au carré de l'épaisseur dudit système et elle est proportionnelle aux coefficients de diffusion des composés à travers ledit système.

La présente invention a également pour objet un dispositif pour l'extraction sélective d'un composé à haute valeur ajoutée, et notamment d'un composé biochimique, à partir d'une solution complexe, du type comprenant une cellule subdivisée en deux compartiments par un système réactif membranaire comportant une structure membranaire portant des catalyseurs enzymatiques qui lui confèrent une activité enzymatique et pourvue sur ses deux faces, de couches barrières propres à empêcher le passage à travers ladite membrane active, de substances autres que le composé à extraire, lequel dispositif est caractérisé en ce que la membrane active est constituée par une couche mince d'un gel approprié et porte un seul catalyseur enzymatique, et en ce que les couches barrières sont des structures qui introduisent une différence de mobilité à travers le système membranaire réactif, entre le composé à extraire, et au moins l'un des autres réactifs présents, autres que le composé à extraire.

Selon un mode de réalisation avantageux du dispositif conforme à la présente invention, celui-ci est caractérisé en ce que la cellule est du type réacteur fermé propice à un fonctionnement en discontinu.

Selon un autre mode de réalisation avantageux du dispositif conforme à la présente invention, celui-ci est caractérisé en ce que la cellule est du type réacteur ouvert à flux continu, propice à un fonctionnement en continu.

Selon un mode de réalisation avantageux de dispositif conforme à la présente invention, le catalyseur enzymatique est retenu dans la membrane qui le porte par la disposition sur chacune des deux faces de ladite membrane, d'un film microporeux.

Selon un autre mode de réalisation avantageux du dispositif conforme à la présente invention, le catalyseur enzymatique est fixé par des liaisons de covalence dans la membrane qui le porte.

Pour la mise en oeuvre de la présente invention, on procède comme suit :

1° - Cas d'une réaction réversible à trois réactifs, catalysée par une enzyme E :

$$A \underset{1}{\overset{2}{\underset{E}{\rightleftarrows}}} C + D \qquad (1)$$

Si les concentrations en A et C sont données, D a une concentration d'équilibre déterminée par la loi d'action de masse :

$$K_{eq} = \frac{[C]_{eq} \times [D]_{eq}}{[A]_{eq}} \quad (2)$$

Si [ D ] est supérieur ou inférieur à la concentration d'équilibre $[D]_{eq}$, on déplace la réaction dans un sens ou dans l'autre, de façon à satisfaire à l'équation (2), c'est-à-dire si $[D] < [D]_{eq}$, à produire D en favorisant la réaction aller $A \rightarrow C + D$, et si $[D] > [D]_{eq}$, à consommer D en favorisant la réaction retour $C + D \rightarrow A$.

Soit toujours la réaction réversible (1) et les concentrations en A, C et D conformément à la figure 1 annexée.

Si on établit un gradient de D au travers de la membrane, $[D_1]$ étant > à $[D]_{eq}$, on déplace à gauche de la membrane la réaction dans le sens 1 : $C \rightarrow A$, et à droite de la membrane, comme $[D_2] < [D]_{eq}$, on la déplace dans le sens 2 $A \rightarrow C$.

Si, de plus, on place de chaque côté de la membrane des "barrières" quasi imperméables à C, conformément à la figure 2 annexée, dans laquelle la membrane est désignée par la référence 6 et les barrières sont désignées par la référence 5, le réactif A, placé à droite de la membrane, y pénètre. Alors, $A \rightarrow C$ (cf figure 1), et C ne peut pas passer au travers de la barrière de droite. Il va donc diffuser vers la barrière de gauche, où alors $C \rightarrow A$ et A passe au travers et se retrouve dans le compartiment de gauche, qui s'enrichit en A : il y a transport actif de A de droite à gauche de la membrane.

2o- Cas d'une réaction réversible à plus de trois réactifs : les concentrations des autres réactifs X, Y, Z, etc, interviennent de la même façon dans la loi d'action de masse pour définir la constante d'équilibre $K_{eq}$. Ces coréactifs doivent être présents dans le système ou tout au moins dans la membrane active. Selon leurs concentrations, à $K_{eq}$ il correspond une concentration d'équilibre $D_{eq}$ du composé dont on veut établir le gradient, les valeurs de $D_1$ et $D_2$ sont alors choisies comme précédemment de part et d'autre de cette valeur $D_{eq}$.

3o- Cas d'une réaction réversible à deux réactifs $A \rightleftarrows C$ : la constante d'équilibre $K_{eq}$ vaut

$$K_{eq} = \frac{[C]_{eq}}{[A]_{eq}} ,$$

, la réaction va être déplacée dans la couche membranaire où $[A] > [A]_{eq}$, en favorisant la réaction aller $A \rightarrow C$, et dans l'autre couche où $[A] < [A]_{eq}$ en favorisant la réaction retour $C \rightarrow A$. En plus du transport du composé A par diffusion, il s'établit dans la membrane une succession destruction-production sélective de ce composé A aboutissant à un transport facilité de A et donc à son extraction de façon sélective.

Par exemple, dans le cas de la réaction :

$$\text{Alcool} + NAD^{+} \xrightleftharpoons{ADH} \text{Aldéhyde} + NADH + H^{+}$$

où la concentration en alcool est fixe et importante, donc ne joue aucun rôle,
ADH désigne l'alcool-déshydrogénase
le NADH est le composé A ci-dessus à transporter
le NAD+ est le composé C bloqué par les barrières
H+ est D, soit le composé dont on impose un gradient.

La membrane est avantageusement une membrane d'agarose gélifié, doublée d'un film microporeux tel qu'un film "Millipore" par exemple, pour empêcher la "fuite" de l'enzyme qui n'est pas fixée dans la membrane par liaison covalente, étant cependant entendu que l'enzyme peut également être fixée à la membrane par liaison covalente.

La "barrière" peut avantageusement être constituée par un polymère non disponible dans le commerce, mis au point dans le laboratoire, partiellement quaternisé, le Q-P(TDAE) , qui présente pour le NAD+ une perméabilité cent fois plus faible que pour le NADH [Q-P(TDAE) ou "partially quaternized poly(thio-1-(N,N-diethylaminomethyl)ethylene)") ; ce composé et sa préparation sont décrits dans un Article paru dans POLYMER JOURNAL, 12, No 2, pages 113-124 (1980)] .

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention, et aux dessins annexés dans lesquels :

Les figures 3 et 5 sont des représentations schématiques, en coupe verticale, d'un dispositif d'extraction sélective d'un composé à haute valeur ajoutée, à l'aide d'un système réactif membranaire monoenzymatique,

conforme à l'invention,
et les figures 4 et 6 représentent des graphiques dans lesquels les courbes 1, 2 et 10 à 13 (figure 4) et 13 à 17 (figure 6) respectivement mesurent les variations de la concentration du NADH dans un dispositif du type représenté à la figure 3.

Il doit être bien entendu, toutefois, que ces exemples de mise en oeuvre, ces dessins et les parties descriptives correspondantes, sont donnés uniquement à titre d'illustration de l'objet de l'invention, dont ils ne constituent en aucune manière une limitation.

## EXEMPLES

### EXEMPLE 1 - Extraction du NADH d'une solution complexe à l'aide d'un système réactif membranaire monoenzymatique de transport actif.

Comme on le sait, les cycles métaboliques classiques tels que le cycle de Krebs, la glycolyse, la fermentation, la chaine respiratoire, utilisent des déshydrogénases pour catalyser l'oxydation du NADH (ou nicotinamide-adénine-dinucléotide, forme réduite) en NAD+ (nicotinamide-adénine-dinucléotide, forme oxydée) , en présence d'un agent oxydant, aussi bien que la réduction du NAD+ en NADH en présence d'un agent réducteur (ou leurs formes phosphorylées).

On sait depuis longtemps que la réduction du NAD+ acidifie le milieu réactionnel en produisant un proton, tandis que l'oxydation du NADH consomme un proton conformément à la réaction réversible :
$NAD^+ + RH_2 \rightleftarrows NADH + H^+ + R$ où $RH_2$ est un réducteur.

L'alcool- déshydrogénase extraite de levure (yADH, EC 1.1.1.1.) catalyse préférentiellement la réaction réversible d'oxydo-réduction du couple éthanol/acétaldéhyde :

$$CH_3CH_2OH + NAD^+ \underset{}{\overset{yADH}{\rightleftarrows}} CH_3CHO + NADH + H^+$$

Alors que le pH optimal de la réaction d'oxydation du NADH, qui consomme un proton, est de l'ordre de 6, le pH optimal de la réaction de réduction de NAD+, qui produit un proton, est de l'ordre de 8,3.

Le fait que la réaction d'oxydation du NADH a lieu dans une zone de pH inférieure à celle dans laquelle se produit la réaction de réduction du NAD+ est utilisé comme suit, conformément à la présente invention :

L'ADH est répartie de façon homogène dans une membrane mince (de l'ordre de 1 mm d'épaisseur), 6, en gel d'agarose à 3,5 % dans l'eau, de telle manière que le gel contient 210 unités (0,6 mg) d'yADH (A 3263 fournie par SIGMA) pour 0,2 ml de gel. La membrane est insérée (cf figure 3) entre deux "barrières" dont chacune est constituée par un film très mince, 5, d'agarose contenant 10 mg de Q-P(TDAE) à 14 %, qui est perméable au NADH et sensiblement imperméable au NAD+. L'yADH peut être fixée à la membrane d'agarose par liaison de covalence à l'aide de glutaraldéhyde, par exemple, ou bien elle peut simplement être répartie dans la membrane sans lui être fixée, auquel cas il est avantageux de retenir l'enzyme dans la membrane 6 à l'aide d'une membrane micro-poreuse (par exemple de type Millipore, de porosité 0,025 µm) 3,4. La membrane composite 3,4,5 a une épaisseur d'environ 0,3 mm. Le système réactif membranaire 3,4,5,6 ainsi formé est mis en place dans une cellule 7 dans laquelle il délimite deux compartiments, le compartiment D (donneur) et le compartiment R (receveur), étanches l'un par rapport à l'autre. Les deux compartiments D et R, contiennent initialement la même solution tamponnée (tampon phosphate 20 mM) de tous les composants nécessaires aux réactions d'oxydo-réduction : éthanol (2 M), acétaldéhyde (1 mM) et NADH (0,5 mM) à l'exception du NAD+. Chacun des compartiments contient 10 ml de liquide pour une surface de membrane en contact avec les solutions de chaque fois 2 $cm^2$. Au temps initial $t = 0$, on ajuste le pH des compartiments D et R respectivement à 7,2 et 8,6 à l'aide d'un pH-stat. Deux cellules à circulation 8,9, d'un spectrophotomètre S permettent de mesurer en continu la densité optique à 340 nm des solutions contenues dans chacun des compartiments D et R, qui varie en fonction de la concentration en NADH dans chacun des deux compartiments.

Les courbes des concentrations du NADH dans les deux compartiments relevées en fonction du temps, sont représentées à la figure 4 annexée : elles révèlent l'existence de deux phases : la première phase, qui correspond à une diminution asymétrique des concentrations de NADH dans les deux compartiments, constitue en fait, la phase de remplissage de la membrane (initialement vide de NADH et de NAD+) ainsi que l'établissement progressif des profils de concentration (H+, NADH, NAD+) à l'intérieur de la membrane.

La seconde phase constitue le transport actif proprement dit caractérisé par le fait que la concentration de NADH augmente dans le compartiment receveur R où la concentration en ions H+ est la plus faible (à pH = 8,6) et diminue dans le compartiment donneur D (à pH = 7,2). Ces deux phases sont représentées par les courbes 1, 2 et 10 à 13 dont la première, 11, figure la courbe de remplissage de la membrane en NAD, tandis que la courbe 12 figure la courbe de dégradation du NADH, et la courbe 13 la somme de ces deux courbes ; les courbes 1 et 2 représentent l'évolution en fonction du temps de la concentration du NADH dans le compartiment donneur à pH 7,2 et l'évolution de cette même concentration en tenant compte de la dégradation non enzymatique du NADH à pH 7,2 (courbe 12), la courbe 10 représente l'évolution en fonction du temps de la concentration du NADH dans le compartiment receveur à pH 8,6.

En effet, en même temps que cette réaction d'oxydoréduction bouclée et en raison de la longue durée de l'expérience, il se produit une dégradation lente, pH-dépendante, du NADH. Cette dégradation est plus importante dans le compartiment où le pH est égal à 7,2.

En tenant compte de cette dégradation à pH 7,2 et pH 8,6 facilement mesurable par la même méthode de

mesure de la densité optique aux mêmes pH, il est facilement vérifié qu'après la phase de remplissage tout le NADH qui quitte le compartiment donneur D se retrouve dans le compartiment receveur R.

L'expérience a été poursuivie jusqu'au temps t = 78 heures pour lequel le compartiment receveur R titre une concentration de 0,73 mM et le compartiment donneur D, une concentration 0,34 mM.

Les "barrières" 5 ont à la fois pour rôle d'empêcher la fuite des molécules d'enzyme hors de la membrane réactive 6, jouant ainsi un rôle de "tamis moléculaire", et de constituer, en raison de leur charge, due à la présence du Q-P(TDAE), partiellement quaternisé à 14 %, une membrane permsélective qui est pratiquement imperméable au NAD+ et perméable au NADH, ce qui explique qualitativement le transport actif du NADH du compartiment D dans le compartiment R.

Quantitativement la simulation numérique du système réactif membranaire monoenzymatique conforme à la présente invention, a été réalisée à l'aide de l'équation de diffusion-réaction :

$$\frac{\partial c_i}{\partial t} = D_i \frac{\partial^2 c_i}{\partial x^2} + R_i\ (c_k)$$

où :
$c_i$ est la concentration de l'espèce i
t la durée
$D_i$ le coefficient de diffusion de i
x les coordonnées dans l'espace du gel et
$R_i$ la vitesse de la réaction enzymatique impliquant i.

L'estimation des profils de concentration des différentes espèces qui traversent la membrane 6 et des variations des concentrations de NADH dans les compartiments D et R, donnée par la simulation numérique, s'est avérée coïncider avec les données expérimentales qui sont représentées à la figure 4.

Pour cette simulation numérique, on a lissé les pH-dépendances réelles des réactions aller et retour par les équations :
$v = \exp(-.1966(pH-6)^2+9,453)$ pour la réaction aller de pH optimal 6,0, et
$v = \exp(-.2747(pH-8,2)^2+7,5)$ pour la réaction retour de pH optimal 8,2.

Les coefficients de diffusion ont été déterminés expérimentalement (exprimés en $dm^2/s$) :

| | couche réactive | barrières |
|---|---|---|
| NADH | $10^{-7}$ | $10^{-8}$ |
| $NAD^+$ | $10^{-7}$ | $3.10^{-11}$ |
| Aldéhyde | $10^{-7}$ | $10^{-8}$ |
| Alcool | $10^{-7}$ | $10^{-8}$ |

Les Km michaeliens ont été choisis en accord avec la littérature :
NAD+ : 0,2 mM, NADH : 0,02 mM,
Alcool : 20 mM ; Aldéhyde : 0,2 mM

L'incrément de temps a été choisi de .04 s.

Les équations aux dérivées partielles de diffusionréaction ont été appliquées aux espèces NAD+, NADH et Aldéhyde. L'enzyme est considérée comme non diffusante et la concentration d'alcool comme constante dans tout le système, en raison de sa valeur très élevée par rapport aux autres concentrations.

EXEMPLE 2

Pour réaliser l'accumulation et la concentration du composé à extraire, le procédé nécessite l'existence d'une réaction réversible entre au moins trois réactifs. De nombreuses réactions enzymatiques étant des réactions à deux réactifs, par exemple celles catalysées par les isomérases, nous avons montré qu'il était néanmoins possible de concentrer l'un de ces réactifs en bloquant la réaction enzymatique complète et en limitant la réaction chimique à la formation réversible du complexe enzyme substrat. Nous obtenons alors le schéma classique E + S⇄ES à trois réactifs E, S et ES. Les deux espèces E et ES sont alors confinées à l'espace intramembranaire par des barrières de porosité et un gradient d'effecteur, tel qu'un gradient de proton, crée le gradient de la forme active de E, seule capable de se lier avec S.

C'est ainsi qui l'alcool déshydrogénase de levure (yADH) peut fixer le NADH par un mécanisme non ordonné :

yADH + NADH⇄yADH - NADH

Nous avons donc illustré ce mode de fonctionnement du procédé basé sur la complexation enzyme-substrat par l'extraction du NADH par l'alcool déshydrogénase, le blocage de la réaction enzymatique complète étant réalisé par l'absence de co-substrat. Ce blocage, dans le cas de réac tions à deux réactifs, peut s'effectuer par addition d'inhibiteurs de la réaction.

Conformément à la présente invention, le système est composé comme suit :
l'yADH est répartie de façon homogène dans une membrane mince (de l'ordre de 1 mm d'épaisseur et 2 $cm^2$ de surface), 6, en gel d'agarose à 3,5 % dans l'eau contenant 0,2 mg d'yADH (soit 0,03 mM de site enzymatique/ml). La membrane est insérée entre deux barrières microporeuses 3 (par exemple du type Millipore de porosité 0,025 µm et d'épaisseur 0,1 mm). Le système réactif membranaire ainsi formé est mis en place dans une cellule 7 (cf figure 5) dans laquelle il délimite deux compartiments, le compartiment D (donneur) et le compartiment R (receveur), étanches l'un par rapport à l'autre. Les deux compartiments contiennent initialement la même solution tamponnée (tampon phosphate 100 mM) de NADH 0,15 mM. Chaque compartiment contient 10 ml de solution. Au temps initial $t = 0$, on ajuste le pH des compartiments D et R respectivement à 7,5 et 8,3 à l'aide d'un pH-stat.

Deux cellules à circulation 8 et 9, d'un spectrophotomètre S, permettent de mesurer en continu la densité optique à 340 nm des solutions contenues dans chacun des compartiments D et R, qui varie proportionnellement à leur concentration en NADH.

Les courbes des concentrations en NADH dans les deux compartiments relevées en fonction du temps, sont représentées à la figure 6 annexée : elles révèlent, comme dans l'exemple 1, l'existence de deux phases : la première, qui correspond à une diminution asymétrique des concentrations en NADH dans les deux compartiments, constitue en fait la phase de remplissage de la membrane (initialement vide de NADH) ainsi que de l'établissement des profils de concentration intramembranaire. Cette phase a duré environ 400 minutes.

La seconde phase constitue le transport actif proprement dit caractérisé par l'augmentation de la concentration en NADH dans le compartiment receveur (pH 8,3) et la poursuite de sa diminution dans le compartiment donneur (pH 7,5). Ces deux évolutions sont représentées par les courbes 14 et 15. Le remplissage de la membrane est représenté par la courbe 16 et la destruction non enzymatique du NADH à pH 7,5 par la courbe 17. Cette dégradation du NADH a déjà été mentionnée dans l'exemple 1.

Au bout d'un temps global de 1000 minutes, l'écart entre les deux compartiments de la concentration en NADH a atteint 0,013 mM.

La simulation numérique sur ordinateur confirme ce type d'expérience.

EXEMPLE 3 - Extraction de glucose à l'aide d'un système membranaire comprenant des barrières chargées.

La concentration du glucose et le blocage du $G_6P$ sont obtenus en utilisant comme barrières un film d'agarose contenant de l'acide polyacrylique qui bloque le $G_6P$ en reproduisant sensiblement les conditions décrites à l'exemple 1 et en utilisant l'hexokinase comme catalyseur.

EXEMPLE 4 - Extraction d'amino-acide à l'aide d'un système membranaire comprenant des barrières de porosité.

En utilisant une membrane de transport actif portant une exopeptidase, associée à des barrières neutres constituées par des films micro-poreux (de type Millipore par exemple), on réalise la synthèse-coupure enzymatique de protéines pour extraire des amino-acides spécifiques, qui passent à travers les films micro-poreux, alors que les peptides à partir desquels sont coupés les amino-acides ne passent pas à travers lesdits films.

Peptide $_n$⇆peptide$_{n-1}$ + amino-acide

EXEMPLE 5 - Extraction d'adénine.

En utilisant une membrane en gel d'agarose portant de l'adénine-phosphoribosyl-transférase, limitée par des barrières hydrophobes, on extrait l'adénine, elle aussi hydrophobe en donnant l'intermédiaire hydrophile AMP.

Les exemples qui précèdent font apparaître que l'invention englobe les réactions enzymatiques réversibles les plus diverses.

Les exemples qui précèdent font apparaître également que l'invention englobe les barrières qui empêchent une des composantes de la réaction de sortir de la membrane, ceci en jouant sur la charge électrique de la barrière, sur sa porosité ou encore sur son hydrophobicité.

De même, l'invention englobe les utilisations de ce procédé dans les réacteurs de type discontinu, aussi bien que dans les réacteurs à flux continu. L'invention s'applique également aux systèmes plurienzymatiques.

**Revendications**

1.- Procédé d'extraction sélective d'un composé à haute valeur ajoutée, et notamment d'un composé biochimique, à partir d'une solution complexe, caractérisé en ce que l'on met en oeuvre pour réaliser

ladite extraction un système réactif membranaire, monoenzymatique, apte à catalyser une réaction réversible, qui est placé en milieu liquide, en ce que l'on crée aux interfaces système membranaire/milieu liquide, des conditions asymétriques à l'aide d'un gradient de potentiel électrochimique de l'un des réactifs, lesquelles conditions asymétriques forcent la réaction réversible à travailler dans un sens d'un côté du système réactif membranaire et dans l'autre sens de l'autre côté, en ce que le composé à extraire effectue, par suite, une boucle de réaction qui le fait passer d'un côté à l'autre de la membrane et en ce que le passage du composé à extraire d'un côté du système réactif membranaire vers l'autre côté et son accumulation de ce côté, sont favorisés par la présence sur les deux faces dudit système d'une couche constituant une barrière spécifique propre à confiner dans l'espace intramembranaire, au moins l'un des composés autres que le composé à extraire.

2.- Procédé selon la revendication 1, caractérisé en ce que la couche constituant une barrière est une structure qui interdit le passage du composé à confiner dans l'espace intramembranaire, les moyens d'interdiction du passage étant choisis en fonction de la nature du composé à confiner.

3.- Procédé selon la revendication 2, caractérisé en ce que lorsque le composé à confiner est chargé, la barrière porte une charge globale de signe opposé.

4.- Procédé selon la revendication 2, caractérisé en ce que lorsque le composé à confiner est une grosse molécule, la barrière comprend un film microporeux.

5.- Procédé selon la revendication 2, caractérisé en ce que lorsque le composé à confiner présente une hydrophobicité donnée, la barrière comprend une substance lui conférant une hydrophobicité de nature opposée.

6.- Procédé selon l'une quelconque des revendications 1 à 5, caractérisé en ce que le rapport des coefficients de diffusion

$$\frac{\text{composé à extraire}}{\text{composé à confiner dans l'espace intramembranaire}} \gg 1$$

7.- Procédé selon l'une quelconque des revendications 1 à 6, caractérisé en ce que lorsque la réaction implique au moins trois réactifs, à savoir un réactif à extraire, au moins un co-réactif et au moins un composé destiné à être confiné dans l'espace intramembranaire défini par le système membranaire, on établit le gradient de potentiel électrochimique du co-réactif ou de l'un des co-réactifs, pour une constante d'équilibre $K_{eq}$ donnée, en ajustant les potentiels électrochimiques à des valeurs différentes dans les compartiments récepteur et donneur définis par le système membranaire, de part et d'autre de ce dernier, de telle façon que le rapport K donné par la loi d'action de masse, dans lequel la concentration du composé à extraire est en dénominateur, soit $K < K_{eq}$ du côté donneur et
$K > K_{eq}$ du côté récepteur.

8.- Procédé selon l'une quelconque des revendications 1 à 7, caractérisé en ce que lorsque la réaction implique des protons, on utilise avantageusement ces protons comme réactif dont on veut établir le gradient, en ajustant les compartiments donneur et récepteur situés de part et d'autre du système membranaire, à des pH différents.

9.- Procédé selon l'une quelconque des revendications 1 à 6, caractérisé en ce que lorsque la réaction implique au moins deux réactifs,à savoir un composé à extraire et un réactif,on forme un complexe entre le composé à extraire et l'enzyme, on confine à la fois l'enzyme et le complexe enzyme-composé dans l'espace intramembranaire et on établit un gradient intermédiaire d'un effecteur approprié, tel, notamment, qu'un gradient de pH, lequel gradient intermédiaire provoque dans l'espace intramembranaire, l'établissement d'un gradient d'enzyme libre active, qui est le gradient de potentiel électrochimique voulu.

10.- Procédé selon l'une quelconque des revendications 1 à 6, caractérisé en ce que dans le cas où la réaction enzymatique implique au moins deux réactifs, du type $A \leftrightarrows B$, on applique le gradient de potentiel chimique au composé que l'on cherche à extraire, lui-même, la source énergétique étant alors constituée par la dissipation de ce gradient pour réaliser un transport facilité qui augmente sélectivement la vitesse de passage à travers la membrane, du composé à extraire.

11.- Procédé selon l'une quelconque des revendications 1 à 6, caractérisé en ce que dans le cas où la réaction enzymatique implique un nombre quelconque de réactifs, on forme et décompose un complexe enzyme-composé à extraire $E + A \leftrightarrows EA$ et on utilise l'enzyme elle-même,sous forme libre ou complexée,comme substance confinée dans l'espace intramembranaire, auquel cas le gradient est obtenu par un gradient d'effecteur et notamment de protons, pour réaliser un transport actif.

12.- Procédé selon la revendication 11, caractérisé en ce que le gradient est obtenu, en variante, en utilisant le composé à extraire lui-même, pour réaliser un transport facilité qui accélère sélectivement la diffusion du composé à extraire.

13.- Dispositif pour l'extraction sélective d'un composé à haute valeur ajoutée, et notamment d'un composé biochimique, à partir d'une solution complexe, du type comprenant une cellule subdivisée en deux compartiments par un système réactif membranaire comportant une structure membranaire portant des catalyseurs enzymatiques qui lui confèrent une activité enzymatique et pourvue, sur ses deux faces, de couches barrières propres à empêcher le passage à travers ladite membrane active, de substances

autres que le composé à extraire, lequel dispositif est caractérisé en ce que la membrane active est constituée par une couche mince d'un gel approprié et porte un seul catalyseur enzymatique, et en ce que les barrières sont des structures qui introduisent une différence de mobilité à travers le système réactif membranaire, entre le composé à extraire et au moins l'un des autres réactifs présents, autres que le composé à extraire.

14.- Dispositif selon la revendication 13, caractérisé en ce que la cellule est du type réacteur fermé propice à un fonctionnement en discontinu.

15.- Dispositif selon la revendication 13, caractérisé en ce que la cellule est du type réacteur ouvert à flux continu, propice à un fonctionnement en continu.

16.- Dispositif selon l'une quelconque des revendications 13 à 15, caractérisé en ce que le catalyseur enzymatique est retenu dans la membrane qui le porte par la disposition sur chacune des deux faces de ladite membrane, d'un film microporeux.

17.- Dispositif selon l'une quelconque des revendications 13 à 15, caractérisé en ce que le catalyseur enzymatique est fixé par des liaisons de covalence dans la membrane qui le porte.

0247931

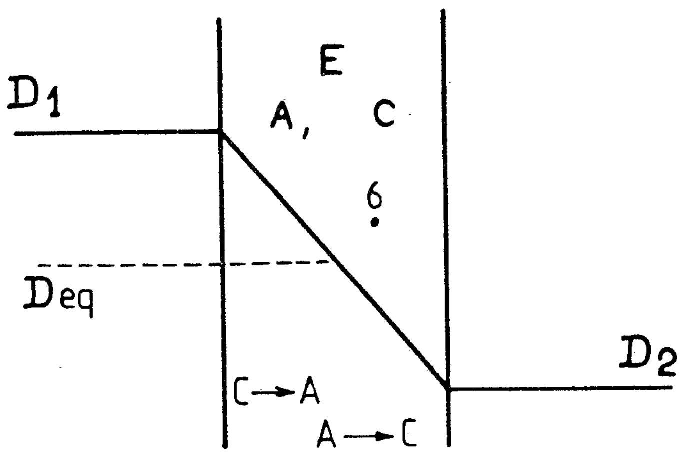


FIG_1

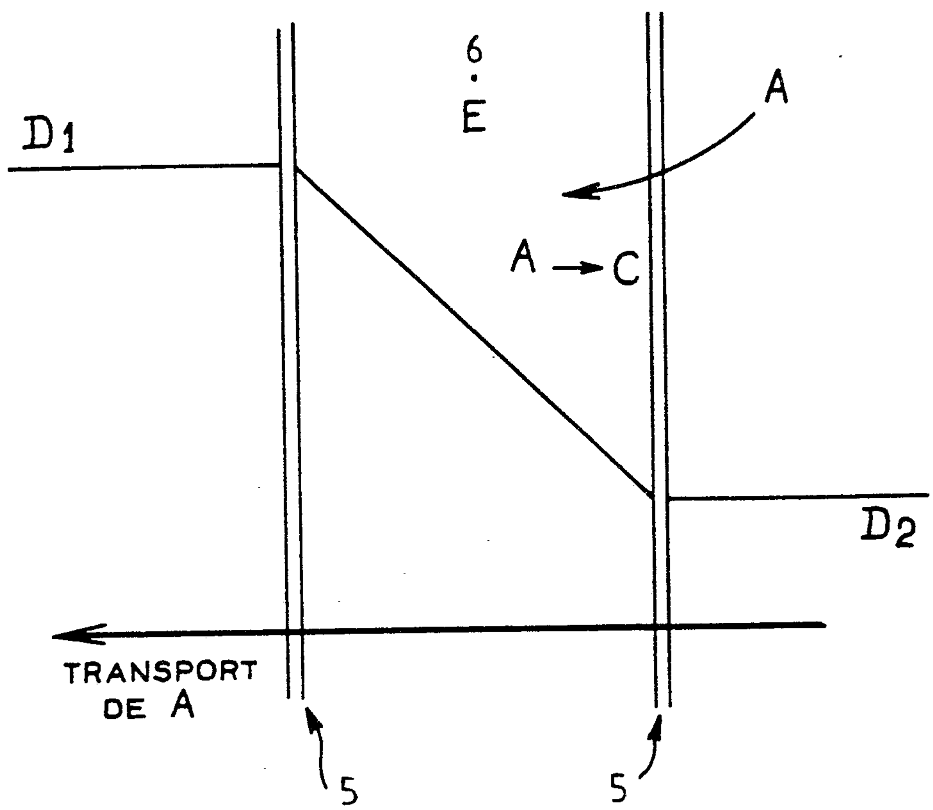


FIG_2

0247931

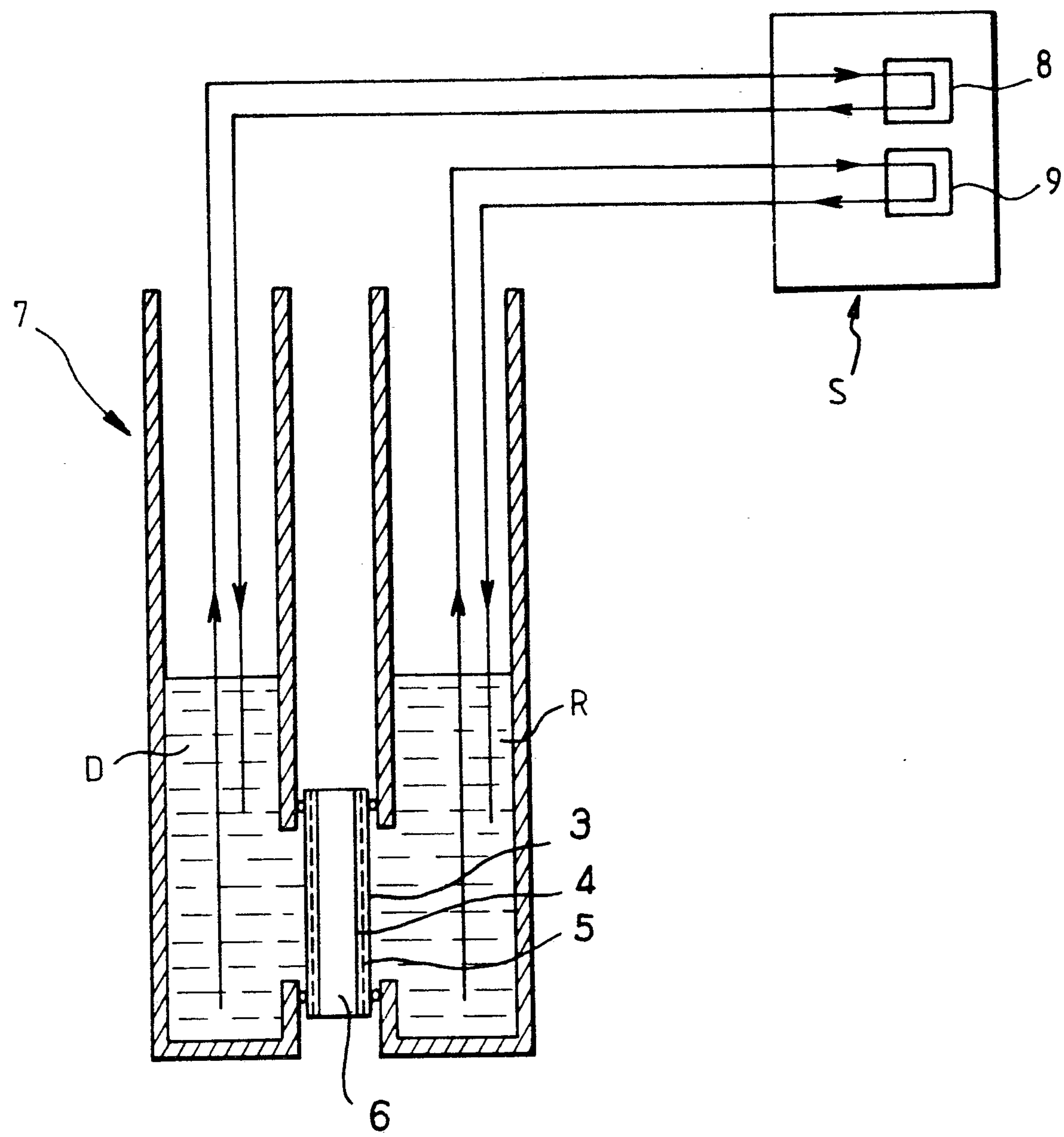


FIG_3

FIG_4

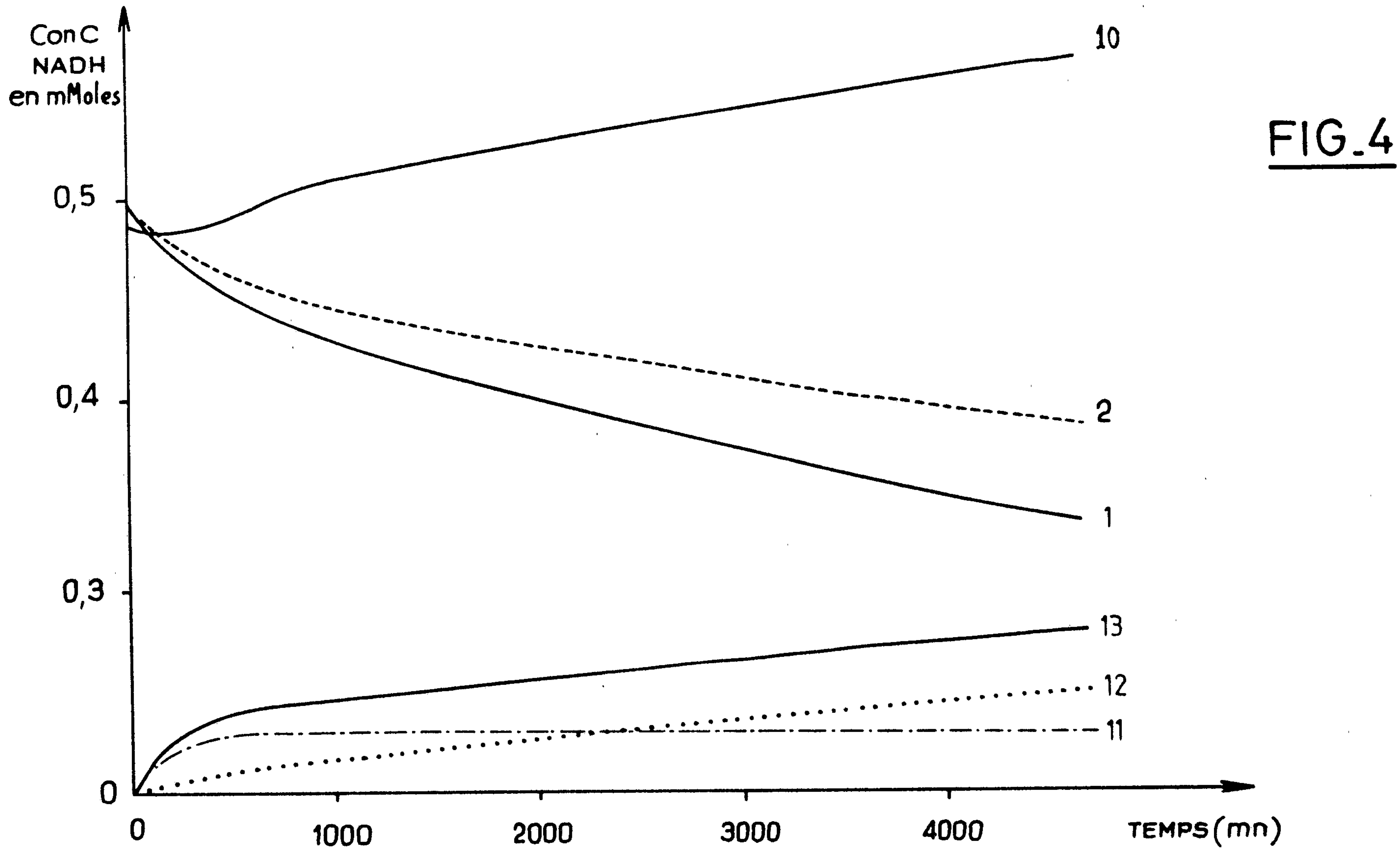
ConC
NADH
en mMoles
0,5
0,4
0,3
0
0
1000
2000
3000
4000
TEMPS (mn)
10
2
1
13
12
11

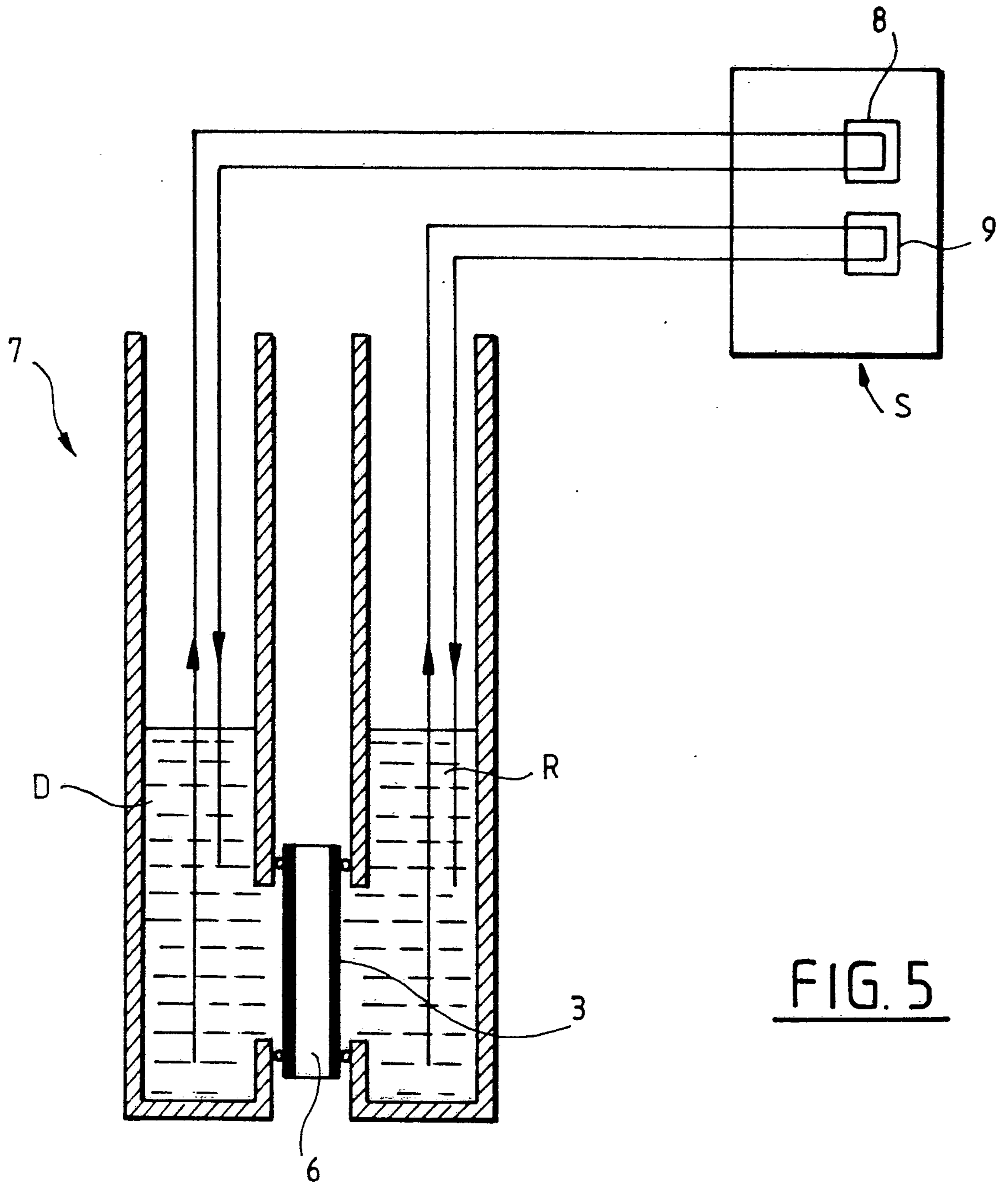


FIG. 5

FIG. 6

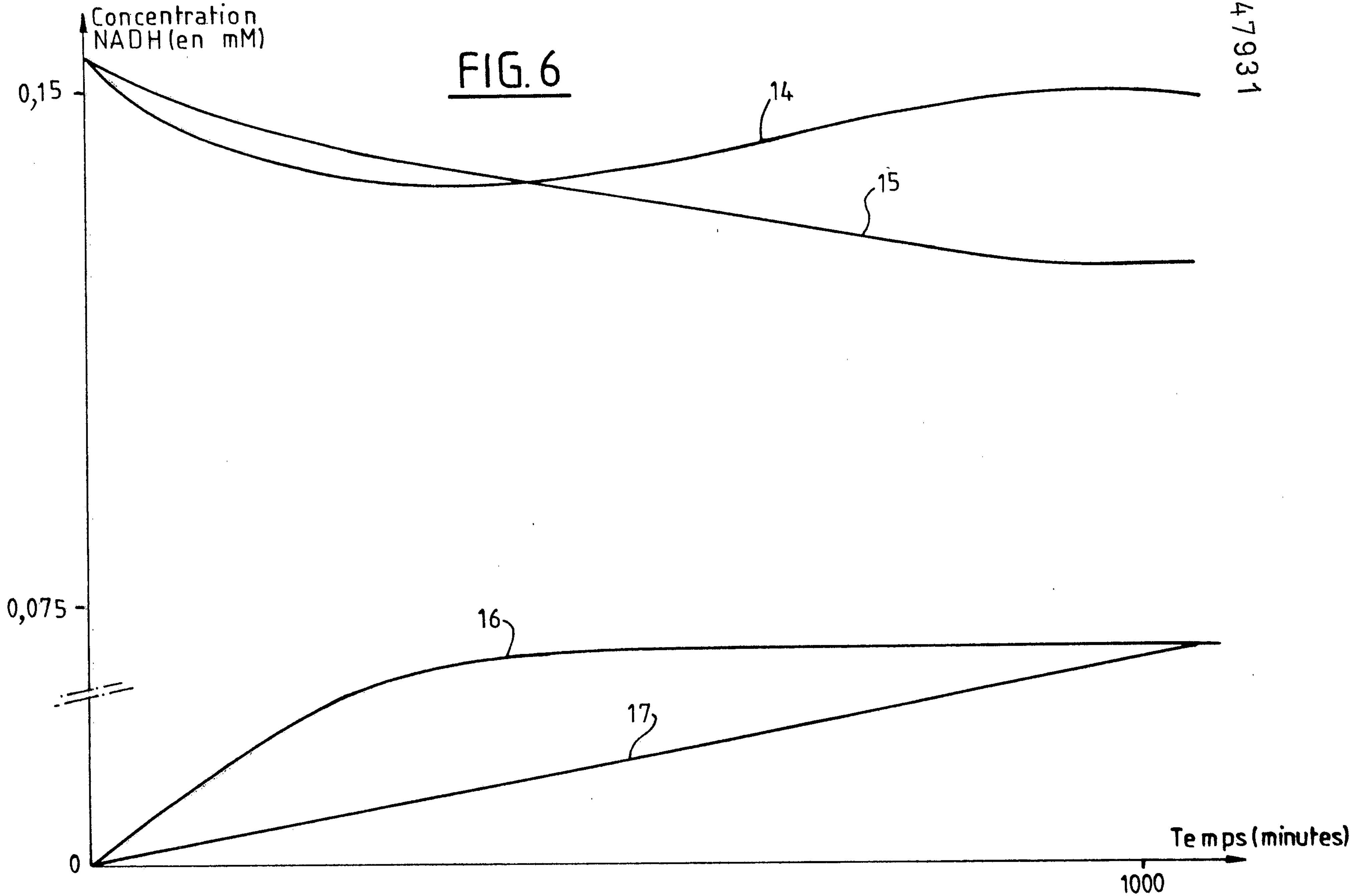

Concentration NADH (en mM)
0,15
0,075
0
14
15
16
17
Temps (minutes)
1000

Office européen des brevets

# RAPPORT DE RECHERCHE EUROPEENNE

Numéro de la demande
EP 87 40 1155

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,A | CHEMICAL ABSTRACTS, vol. 93, no. 21, novembre 1980, page 214, résumé no. 199369a, Columbus, Ohio, US; E. SELEGNY et al.: "Two enzyme active transport in vitro with pH induced asymmetrical functional structures: I. The model and its analytical treatment", & BIOPHYS. CHEM. 1980, 12(1), 93-106 * En entier * --- | 1 | C 12 M 1/40 |
| A | CHEMICAL ABSTRACTS, vol. 99, no. 17, octobre 1983, pages 237-238, résumé no. 135631w, Columbus, Ohio, US; J.C. VINCENT et al.: "Ion transport by asymmetrical functional membrane model", & STUD. PHYS. THEOR. CHEM. 1983, 24(Phys. Chem. Transmembr. Ion Motions) 123-8 * En entier * --- -/- | 1 | DOMAINES TECHNIQUES RECHERCHES (Int. Cl. 3)<br>C 12 M<br>C 12 P |

Le présent rapport de recherche a été établi pour toutes les revendications

| Lieu de la recherche | Date d'achèvement de la recherche | Examinateur |
|---|---|---|
| LA HAYE | 03-08-1987 | WIESER, M.R.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

**RAPPORT DE RECHERCHE EUROPEENNE**

Numéro de la demande

EP 87 40 1155



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | CHEMICAL ABSTRACTS, vol. 90, no. 13, mars 1979, page 148, résumé no. 98692h, Columbus, Ohio, US; J.C. VINCENT et al.: "Active transport of glucose "in vitro" illustration of asymmetrical functional structures and of allotopia", & COLLOQ. INT. C. N. R. S. 1976 (Pub. 1977) 258(Echanges Ioniques Transmembr. Veg.), 147-53<br>* En entier *<br>--- | 1 | |
| X | GB-A-2 164 663 (KAO CO.)<br>* Revendications 1,2; figures 1,2 *<br>----- | 13,15, 16 | |

DOMAINES TECHNIQUES RECHERCHES (Int. Cl. 3)

Le présent rapport de recherche a été établi pour toutes les revendications

| Lieu de la recherche | Date d'achèvement de la recherche | Examinateur |
|---|---|---|
| LA HAYE | 03-08-1987 | WIESER, M.R.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant